(19) Europäisches Patentamt / European Patent Office / Office européen des brevets

(11) **EP 3 519 056 B1**

(12) **FASCICULE DE BREVET EUROPEEN**

(45) Date de publication et mention de la délivrance du brevet:
**01.07.2026 Bulletin 2026/27**

(21) Numéro de dépôt: **17784358.8**

(22) Date de dépôt: **20.09.2017**

(51) Classification Internationale des Brevets (IPC):
*A61Q 5/00* (2006.01)    *A01G 33/00* (2006.01)
*A61Q 19/00* (2006.01)    *A61K 8/97* (2017.01)
*A61K 36/02* (2006.01)    *A61K 36/04* (2006.01)
*C12N 1/12* (2026.01)    *A61P 17/08* (2006.01)
*A61P 17/04* (2006.01)    *A61K 8/9706* (2017.01)
*A61P 17/00* (2006.01)    *C12P 1/00* (2006.01)

(52) Classification Coopérative des Brevets (CPC):
**A61Q 19/00; A61K 8/9706; A61K 36/02;
A61K 36/04; A61P 17/00; A61P 17/04;
A61P 17/08; A61Q 5/00; C12N 1/12; C12P 1/00;**
A61K 2236/00

(86) Numéro de dépôt international:
**PCT/FR2017/052521**

(87) Numéro de publication internationale:
**WO 2018/060567 (05.04.2018 Gazette 2018/14)**

(54) **PROCÉDÉ POUR EMPÊCHER OU RALENTIR L'APPARITION DES SIGNES INESTHÉTIQUES GÉNÉRÉS PAR LES AGENTS POLLUANTS PRÉSENTS DANS L'ATMOSPHÈRE SUR LA PEAU, LE CUIR CHEVELU, LES CHEVEUX OU LES MUQUEUSES**

VERFAHREN ZUM VERHINDERN ODER VERHINDERN DES ERSCHEINENS VON INESSHETISCHEN ZEICHEN, DIE DURCH DIE IN DER ATMOSPHÄRE VORHANDENEN SCHADSTOFFE AN HAUT, SKALP, HAAR ODER MUCOSES ERZEUGT WERDEN

METHOD FOR PREVENTING OR SLOWING THE APPEARANCE OF INESTHETIC SIGNS GENERATED BY POLLUTANTS IN THE ATMOSPHERE ON SKIN, SCALP, HAIR OR MUCOSES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.09.2016 FR 1659394**

(43) Date de publication de la demande:
**07.08.2019 Bulletin 2019/32**

(73) Titulaire: **Société d'Exploitation de Produits pour les Industries Chimiques SEPPIC 75321 Paris Cedex 7 (FR)**

(72) Inventeurs:
• **BERGE, Armelle 81100 Castres (FR)**
• **CATTUZZATO, Laetitia 81100 Castres (FR)**
• **LE GELEBART, Erwan 22260 Ploubazlanec (FR)**

(74) Mandataire: **Air Liquide L'Air Liquide S.A. Direction de la Propriété Intellectuelle 75, Quai d'Orsay 75321 Paris Cedex 07 (FR)**

(56) Documents cités:
CN-A- 102 475 664    CN-A- 103 931 482
DE-U1- 202007 012 678    JP-A- 2005 047 910
US-A1- 2006 198 800    US-A1- 2008 107 679
US-B1- 6 572 868

- CENTERCHEM: "APT(TM)-GPF - product info", 1 January 2013 (2013-01-01), XP055358702, Retrieved from the Internet <URL:http://www. centerchem.com/Products/DownloadFile.aspx? FileID=6320> [retrieved on 20170324]
- DAWES C J ET AL: "BRANCH, MICROPROPAGULE AND TISSUE CULTURE OF THE RED ALGAE EUCHEUMADENTICULATUM AND KAPPAPHYCUS ALVAREZII FARMED IN THE PHILIPPINES", JOURNAL OF APPLIED PHYCOLOGY, KLUWER, DORDRECHT, NL, vol. 3, no. 3, 1 January 1971 (1971-01-01), pages 247 - 257, XP000993577, ISSN: 0921-8971
- MYOUNGHOON MOON ET AL: "Isolation and characterization of thermostable phycocyanin from Galdieria sulphuraria", KOREAN JOURNAL OF CHEMICAL ENGINEERING, vol. 31, no. 3, 15 January 2014 (2014-01-15), pages 490 - 495, XP055146911, ISSN: 0256-1115, DOI: 10.1007/ s11814-013-0239-9
- STEGENGA H ET AL: "REMARKS ON THE AUDOUINELLA MICROSCOPICA (NÄG.) WOELKERLING COMPLEX, WITH A BRIEF SURVEY OF THE GENUS CHROMASTRUM PAPENFUSS (RHODOPHYTA, NEMALIALES)", ACTA BOTANICA NEERLANDICA, BLACKWELL SCIENTIFIC PUBLICATIONS, OXFORD, GB, vol. 28, no. 4-5, 1 January 1979 (1979-01-01), pages 289 - 312, XP002752335, ISSN: 0044-5983, [retrieved on 20150422], DOI: 10.1111/ J.1438-8677.1979.TB00355.X
- CRUZ-URIBE O ET AL: "UPTAKE AND BIOTRANSFORMATIONOF 2,4,6-TRINITROTOLUENE (TNT) BY MICROPLANTLET SUSPENSION CULTURE OF THE MARINE RED MACROALGA PORTIERIA HORNEMANNII", BIOTECHNOLOGY AND BIOENGINEERING, WILEY ETC, vol. 93, no. 3, 20 February 2006 (2006-02-20), pages 401 - 412, XP001241282, ISSN: 0006-3592
- CHATO-SALVADOR RONELIE C ET AL: "Non-enzymatic isolation of somatic cells from Kappaphycus spp. and Eucheuma denticulatum (Solieriaceae, Rhodophyta)", EUROPEAN JOURNAL OF PHYCOLOGY, CAMBRIDGE UNIVERSITY PRESS, CAMBRIDGE, DE, vol. 49, no. 4, 1 November 2014 (2014-11-01), pages 486 - 492, XP002752336, ISSN: 0967-0262, [retrieved on 20141114], DOI: 10.1080/ 09670262.2014.977963

**Description**

La présente invention a pour objet l'utilisation d'un extrait de biomasse unialgale de cellules de macro-algues pluricellulaires de petite taille comme agent anti-pollution pour protéger la peau humaine des effets inesthétiques de la pollution , ainsi qu'un procédé de traitement cosmétique de la peau humaine visant à prévenir des effets inesthétiques de la pollution par application d'une composition topique comprenant un extrait de biomasse unialgale de cellules de macro-algues pluricellulaires de petite taille.

Les effets de la pollution sur la santé des humains constituent des problèmes de santé rencontrés à l'échelle du globe terrestre. En effet, les milieux urbains sont régulièrement exposés à différents polluants aériens et atmosphériques, dont l'action peut se révéler néfaste pour la santé des êtres humains, en détériorant le fonctionnement de certains organes comme les poumons et les yeux par exemple. En constituant une barrière entre le corps humain et l'environnement extérieur, les matières kératiniques constituant le corps humain, et plus particulièrement la peau, les muqueuses et les cheveux, sont en contact avec ces polluants aériens et atmosphériques, conduisant à des effets externes visibles caractérisés par l'apparition et l'accroissement des rides, par un teint terne, par un manque d'uniformité du teint (phénomène de dyschromie), par l'apparition et l'accroissement de tâches pigmentaires (phénomène d'hyper-pigmentation) ou encore par une modification et une altération de l'efficacité de la barrière cutanée.

Les effets systémiques de l'exposition à différents types de polluants sont décrits dans la littérature, et les effets au niveau cutané sont de plus en plus étudiés. Parmi les polluants les plus nocifs, on peut citer les gaz toxiques tels que le mono-oxyde de carbone, les oxydes d'azote tel que le dioxyde d'azote, les oxydes de soufre tel que le dioxyde de soufre, l'ozone ; les métaux lourds comme par exemple le cobalt, le mercure, le cadmium et le nickel, les polluants intérieurs comme par exemple les composés organiques volatils (ou C.O.V.) et la fumée de cigarette, les particules atmosphériques qui sont des solides microscopiques en suspension dans l'atmosphère terrestre comme par exemple les microparticules identifiées sous les acronymes PM2.5 et PM10 [E. Drakaki, C. Dessinioti and C. V. Antoniou, Frontiers in Environmental Science, 2, 1-6 (2014*)*].

Ces polluants sont connus pour engendrer des effets négatifs externes visibles sur la peau, conduisant ou participant à l'apparition des rides, d'un teint terne, d'un manque d'uniformité du teint (phénomène de dyschromie), de tâches pigmentaires et également de désordres cutanés tels que le psoriasis et la dermatite atopique [MA Lefebvre, DM Pham, B Boussouira, D Bernard, C Camus and QL Nguyen, International Journal of Cosmetic Science, 37, 329-38 (2015*)]*, [MA Lefebvre, DM Pham, B Boussouira, H Qiu, C Ye, X Long, R Chen, W Gu, A Laurent and QL Nguyen, International Journal of Cosmetic Science, 38, 217-23 (2016*)*], [A. Vierkötter, Hautarzt, 61, 538-9 (2010*)*], [YS Yang, HK Lim, KK Hong, MK Shin, JW Lee, SW Lee and NI Kim, Annals of Dermatology, 26, 11-16 (2014*)*].

A ce jour, quatre composantes biologiques sont supposées intervenir dans l'apparition de ces effets négatifs externes visibles et de ces désordres [S. E. Mancebo and S. Q. Wang, Journal of European Academy of Dermatology and Venerology, 29, 2326-2332 (2015*)]*, [ND Magnani, XM Muresan, G Belmonte, F Cervellati, C Sticozzi, A Pecorelli, C Miracco, T Marchini, P Evelson and G Valacchi, Toxicological Sciences, 149, 227-36 (2016*)*] :

- La composante oxydante : les agents polluants entraineraient une production accrue de radicaux libres par les cellules cutanées, conduisant à la génération de protéines carbonylées, de lipides peroxydés ou encore à l'apparition de dommages à l'ADN, et en parallèle, une diminution des systèmes anti-oxydants enzymatiques et non enzymatiques sont observée ;
- La composante inflammatoire : les effets des agents polluants induiraient une induction d'une cascade de réactions inflammatoires ;
- L'activation d'un récepteur spécifique aux hydrocarbones (AhR) ;
- L'altération de la microflore cutanée ;

Ces voies sont généralement activées de façon synergique par la combinaison des différents agents polluants.

Il apparait clairement qu'il existe une demande de plus en plus importante pour mettre au point et préparer des compositions topiques comprenant des substances et compositions chimiques, permettant de traiter et de prévenir les effets inesthétiques des agents polluants sur la peau humaine. Lesdites substances et compositions chimiques peuvent agir selon différentes approches :

- Par protection de la peau de façon mécanique, c'est-à-dire en l'isolant des agents polluants ou, de façon plus réaliste, en limitant son exposition auxdits agents polluants par l'application sur la peau d'un matériau « barrière », par exemple une composition topique possédant un effet filmogène ;
- Par protection de la peau de façon chimique, c'est-à-dire en appliquant sur la peau une ou plusieurs substances ou compositions chimiques qui jouent le rôle d'une barrière chimique comme par exemple des substances ou compositions chimiques possédant des propriétés anti-oxydantes, qui complètent l'action des défenses anti-oxydantes intrinsèquement présentes sur la peau, et/ou des propriétés chélatantes des métaux lourds, qui sont susceptible de capter et de piéger lesdits agents polluants, et plus particulièrement les métaux lourds, et par conséquent diminuer ou éliminer l'effet oxydatif desdits agents polluants sur la peau.

- Par protection de la peau de façon biologique, c'est-à-dire en appliquant sur la peau une ou plusieurs substances ou compositions chimiques qui agissent sur les cellules de l'épiderme de la peau pour augmenter leur capacité à diminuer ou éliminer les effets nocifs des agents polluants, comme par exemple diminuer ou éliminer les effets oxydants ou inflammatoires desdits agents polluants.

Parmi les substances et les compositions chimiques susceptibles d'être incorporées dans des compositions cosmétiques à usage topique pour protéger la peau de façon chimique telle que décrite précédemment, on peut citer les agents chélatants comme par exemple l'acide éthylène diamine tétraacétique (ou EDTA), le sel pentasodique d'éthylènediamine tétraméthylène phosphonique, et la N,N'-bis-(3,4,5-triméthoxybenzyl)éthylène diamine ou l'un de ses sels, complexes métalliques ou ester ; l'acide phytique, les extraits de thé et plus particulièrement les extraits de thé vert ; les extraits de Jacinthe d'eau (Eichornia crassipes); et la fraction hydrosoluble de mais commercialisée par la société SOLABIA sous la dénomination commerciale Phytovityl™.

Pour lutter contre ces effets négatifs des agents polluants sur les surfaces kératiniques du corps humain, la demande de brevet européen publiée sous le numéro EP 557 042 A1 divulgue l'utilisation de metallothionéines contre l'effet des métaux lourds et des shingolipides pour protéger plus particulièrement la peau et les cheveux.

La demande de brevet européen, publiée sous le numéro EP 1 230 914 A1, décrit l'utilisation d'un amidon, natif ou modifié, notamment choisi parmi les amidons de maïs, de riz, de manioc, de pomme de terre, de blé, de sorgho, de pois, ainsi que de l'association desdits amidons avec une gomme de silicone, pour protéger les matières kératiniques humaines des effets inesthétiques des agents polluants, et plus particulièrement des agents polluants se présentant sous la forme de particules atmosphériques, d'une façon mécanique.

Les ingrédients d'origine marine présentent un intérêt pour les produits finis cosmétiques de part leur origine naturelle, mais peuvent poser des problèmes d'odeur, de stabilité, d'efficacité, de difficulté et de non reproductibilité de formes galéniques formulées, limitant leur utilisation. La ressource marine est extrêmement grande, et pourtant tout un pan de la diversité marine reste inexploré et/ou inexploité.

Les algues sont des organismes photosynthétiques chlorophylliens vivants dans l'eau ou des milieux très humides. Elles peuvent se développer dans l'eau de mer, l'eau douce, l'eau saumâtre, dans des milieux stagnants, battus ou turbulents. Les algues peuvent être unicellulaires ou pluricellulaires, elles peuvent être brunes, vertes ou rouges et sont classées en fonction de caractéristiques d'ordre cytologique et biochimique. Ces organismes jouent un rôle important à l'échelle globale car ils constituent la base des réseaux trophiques et ils sont impliqués dans la production de l'oxygène de l'atmosphère et dans la fixation du dioxyde de carbone.

Les macro-algues sont des algues pluricellulaires eucaryotes, le plus souvent visibles à l'oeil nu et souvent qualifiées de macrophytes. Elles peuvent se reproduire de manière sexuée ou asexuée ; chez certaines espèces ces deux modes de reproduction se succèdent au cours du cycle de vie. Le cycle de vie peut comprendre une génération, une alternance de deux générations ou de trois générations. On parlera respectivement de cycles monogénétique, digénétique ou trigénétique. En fonction du type de cycle de vie, l'espèce peut se présenter sous forme de gamétophytes, de sporophytes, de carposporophyte ou de tétrasporophyte. Au cours d'un cycle, ces différentes formes de vie peuvent être de mêmes morphologies donc isomorphes, soit de morphologies différentes donc hétéromorphes. La différence de morphologie peut être très significative, rendant impossible le rattachement de deux formes de vie à la même espèce pour une personne non avertie.

Cette différence peut également être marquée par la taille de l'algue, une forme de vie peut être macroscopique et une autre forme de vie peut être microscopique. Par exemple le sporophyte d'*Undaria pinnatifida* mesure près d'un mètre alors que le gamétophyte de cette algue mesure quelques dizaines de micromètres. Il existe environ deux mille espèces d'algues brunes, sept mille espèces algues rouges et mille sept cents espèces d'algues vertes.

Les macroalgues mesurent de quelques dizaines de micromètres dans le cas des algues du genre *Acrochaetium* à une centaine de mètres pour l'espèce *Macrocystis pyrifera.*

La classe des algues rouges, encore appelée Floridéophycée fait partie de l'embranchement des Rhodophytes. La classe des algues rouges Floridéophycée comprend les ordres *Acrochaetiales, Acrosymphytales, Ahnfeltiales, Balbianiales, Balliales, Batrachospermales, Bonnemaisoniales, Ceramiales, Colaconematales, Corallinales, Entwisleiales, Florideophyceae incertae sedis, Gelidiales, Gigartinales, Gracilariales, Halymeniales, Hildenbrandiales, Nemaliales, Nemastomatales, Palmariales, Peyssonneliales, Pihiellales, Plocamiales, Rhodachlyales, Rhodogorgonales, Rhodymeniales, Sebdeniales, Sporolithales* et *Thoreales.*

Les algues rouges contiennent des pigments rencontrés chez les autres végétaux, tels de la chlorophylle a et des caroténoïdes, mais leur originalité consiste dans la présence de phycobiliprotéines : l'allophycocyanine (bleu),la phycocyanine (bleu) et la phycoérythrine qui donne la couleur rouge. L'organisation des chloroplastes différencie les algues rouges des Glaucophytes et des Cyanobactéries.

La pigmentation de l'algue rouge dépend en partie de la longueur d'onde de la lumière qui atteint l'algue. En profondeur, les algues rouges accumulent une grande quantité de phycoérythrine, pigment qui peut absorber la lumière à cette profondeur. En surface, le ratio du pigment rouge diminue, par rapport à la chlorophylle et aux autre phycobiliprotéines, elles deviennent plus vertes malgré leur appellation ; on parle d'adaptation chromatique.

Les algues rouges, notamment du genre Gelidium et du genre Palmaria, sont très utilisées en cosmétique, pour diverses applications. La demande de brevet coréen publiée sous le numéro KR101409764, décrit l'utilisation d'un extrait de *Gelidium amansii,* obtenu à partir d'un procédé de préparation par fermentation lactique, comme ingrédients anti-rides, pour les industries de la cosmétique et de la nutrition.

La demande internationale, publiée sous le numéro WO2013178965 A2, décrit l'utilisation d'extraits d'algues, notamment d'algues rouges telles que *Pterocladia capillacea* et *Palmaria palmata,* comme ingrédients activateurs de la mitophagie, conduisant à un effet détoxifiant.

La demande de brevet français, publiée sous le numéro FR 2 911 278 A1, décrit l'utilisation d'un extrait de l'algue rouge *Palmaria palmata,* obtenu par la mise en œuvre d'un procédé enzymatique, suivi d'étapes de filtration, de décantation, de centrifugation, puis de désactivation des enzymes résiduelles par chauffage, de décoloration, de concentration par la mise en œuvre de techniques connues de l'homme du métier, pour obtenir un extrait riche en sucres possédant des propriétés dépigmentantes de la peau humaine, à destination de l'industrie cosmétique.

Le brevet Coréen, publié sous le numéro KR 10 112 8591 B1, décrit l'utilisation d'extraits des algues rouges *Porphyra tenera* et *Gelidium amansii* dans une composition cosmétique à usage topique en tant qu'agent destiné à protéger la peau humaine des effets néfastes et inesthétique des rayonnements ultra-violets.

La demande de brevet américain publiée sous le numéro US 2006/198800 A1 divulgue notamment l'utilisation de l'algue rouge *Ahnfeltia Concinna,* dans des formulations cosmétiques antirides à appliquer sur la peau.

On peut ainsi citer l'extrait de l'algue brune *Ascophyllum nodosum,* commercialisé par la société Algues & Mer sous le nom de marque Invicity™, décrit comme renforçant la fonction barrière de la peau humaine, en diminuant l'activité des agents polluants de type hydrocarbures (AhR). On peut également citer l'extrait de l'algue rouge *Asparagopsis armata,* commercialisé par la société Algues & Mer sous le nom de marque Ysaline™, décrit comme permettant d'éliminer une partie de la flore bactérienne et fongique cutané.

Parmi les dix mille espèces différentes de macroalgues, une centaine d'entre elles seulement présentent aujourd'hui un intérêt économique. C'est ainsi que certaines macroalgues alimentaires, algues productrices d'hydrocolloïdes et algues productrices de molécules à activité biologique ont fait l'objet de recherche extensive pour mieux comprendre leur physiologie, leur métabolisme et leur reproduction.

Les algues utilisées en alimentation humaine comme *Undaria pinnatifida* plus connue sous le nom de Wakame, *Laminaria japonica* également dénommée Kombu, *Porphyra yezoensis* aussi dénommée Nori et *Enteromorpha intestinalis* aussi dénommée Ao Nori ont été étudiées pour maîtriser leur culture.

Les hydrocolloïdes produits par les algues comme *Chondrus crispus, Kappaphycus spp.* et *Eucheuma denticulatum* sont principalement utilisés comme épaississants et gélifiants dans les produits alimentaires et cosmétiques. Les biomasses produisant ces polymères présentent un intérêt économique certain [Ronelie C. et al. in:« Non-enzymatic isolation of somatic cells from Kappaphycus spp. and Eucheuma denticulatum (Solieriaceae, Rhodophyta) » ; Eur. J. Phycol. (2014), 49(4): 486-492]. A cet égard, Clinton J. Dawes et al divulgue un procédé d'obtention d'une biomasse unialgale de cellules d'algues rouges *Eucheuma denticulatum* et de *Kappaphycus alvarezii;* à partir d'un échantillon prélevé dans le milieu naturel, sa mise en culture pour récolter la biomasse [Clinton J. Dawes et al in: « Branch, micropropagule and tissue culture of the red algae Eucheuma denticulatum and Kappaphycus alvarezii farmed in the Philippines », Jouanl of applied Phycology 3 : 247-257, 1991], Il en de même pour Myounghoon et al. dans le cas de l'algue rouge *Galdieria sulphuraria* [Myounghoon et al. in : « Isolation and charactrization of thermostable phycocyanin from Galdieria sulphuraria », Korean J. Chem. Eng., 31(3), 490-495].

La culture en bioréacteur de l'algue verte *Acrosiphonia coalita,* l'algue brune *Laminaria saccharina* et des algues rouges *Agardhiella subulata, Ochtodes secundiramea, Portieria* **hornemannii** a été étudiée dans le but de synthétiser des molécules à activité biologique [Gregory L. Rorrer et al. Production of bioactive metabolites by cell and tissue cultures of marine macroalgae in bioreactor systems. Plant Cell and Tissue Culture for the Production of Food Ingredients, edited by Fu et al. Kluwer Academic / Plenum Publishers, New York, 1999].

Certaines d'entre elles sont fondées sur la connaissance et sur la maîtrise du cycle de reproduction de la macroalgue. Par exemple, chez les laminariales on peut observer l'alternance entre un sporophyte diploïde développé en thalle macroscopique et des gamétophytes mâles et femelles haploïdes microscopiques. Les sporophytes matures fertile produisent des spores nageantes qui se déposent sur un substrat solide et qui donnent naissance à des gamétophytes. L'étude de leur cycle de vie comme celui *d'Undaria pinnatifida* plus connue sous le nom de Wakame ou de *Laminaria japonica* appelé Kombu a permis de développer la culture de gamétophytes, forme de vie microscopique de ces algues pour ensemencer des supports sur lesquels se développeront des sporophytes macroscopiques valorisables en alimentation humaine. La culture de ces algues se fait d'abord en laboratoire pour la culture de la forme de vie microscopique et puis dans le milieu naturel pour produire la forme de vie macroscopique qui pourra être consommée. Le développement de la culture du sporophyte des algues brunes de l'ordre des laminariales telles qu'*Undaria pinnatifida* dans les années 1980 a conduit au développement d'une technique de culture de gamétophyte dite en « free living », qui consiste à récolter des sporophytes matures, provoquer la sporulation, capter les spores, former des gamétophytes puis les cultiver dans le but de créer des gamètes qui, après fécondation, donneront naissance à de nouveaux sporophytes (R. Perez *et al.,* 1984).

Le développement de la culture du sporophyte de *Laminaria saccharina* a aussi nécessité le développement d'une méthode de culture de son gamétophyte, de manière semblable aux cultures *d'Undaria pinnatifida* [C. Zhi, G. L. Rorrer. Photolithotrophic cultivation of Laminaria saccharina gametophyte cells in a bubble-column bioreactor [Enzyme and Microbial Technology. Volume 18, Issue 4, March 1996, Pages 291-299].

De même à des fins d'études expérimentales, H. Stegenga et al. ont isolé par culture d'échantillons de *Chromastrum moniliforme,* des gamétophytes et des tétrasporophytes de ces algues [H. Stegenga et al. dans : « Remarks on the Audouinella microscopica (NÄG.) Woekerling complex, with a brief survey of the genus chromastrum papenfuss (Rhodophyta, Nemaliales)", Acta Bot. Neerl. 28(4/5), August 1979, p. 289-311].

La demande de brevet chinois publiée sous le numéro CN 103858745 A, divulgue le développement de cultures artificielles de *Sytosiphon lomentaria* en maîtrisant les étapes de différenciation du plasma germinatif de l'algue, pour produire un sporange uniloculaire mature, **puis provoquer** sa sporulation dans le but d'ensemencer des supports de culture en vue de produire en mer, de la biomasse de *Sytosiphon lomentaria* macroscopique.

La demande brevet chinois publiée sous le numéro CN 103931482 A, divulgue une méthode d'obtention de thalle du gamétophyte de l'algue rouge *Porphyra yezoensis,* qui est utilisé en alimentation humaine, notamment pour la confection de makis, impliquant d'abord la culture « *in vitro* » de la phase conchocelis de l'algue afin de produire des conchospores qui se fixeront sur un support de culture pour permettre la croissance de thalles en mer.

D'autres méthodes impliquent l'induction de cals à partir d'explants d'algues rouges, lesdits cals entraînant le développement de plantules qui sont ensuite cultivées en bioréacteur. On peut citer dans ce cadre, les travaux de Ronélie et al. [Ronelie C. et al. Nonenzymatic isolation of somatic cells from Kappaphycus spp. and Eucheuma denticulatum (Solieriaceae, Rhodophyta), Eur. J. Phycol. (2014), 49(4): 486-492] ; ceux de J. Munoz [J. Munoz. Use of plant growth regulators in micropropagation of Kappaphycus alvarezii (Doty) in airlift bioreactors. J Appl Phycol (2006) 18:209-218], ou ceux de Maliakal et al. [S. Maliakal, D. Cheney. Halogenated monoterpenes production in regenerated plantlet cultures of Ochtodes secundiramea. J. Phycol. 37, 1010-1019 (2001)].

D'autres méthodes passent par la production de protoplastes à partir d'un thalle dans le but de reformer de nouveaux thalles, en utilisant, comme proposé par Rusig et al. dans le cas *d'Enteromorpha intestinalis,* algue utilisée en alimentation humaine et animale, un mélange enzymatique contenant de la cellulase et des enzymes d'aplysie permet de digérer la paroi des cellules du thalle de l'algue. Les cellules dont la paroi est digérée s'appelant protoplastes [A Rusig & J. Cosson, Plant regeneration from protoplasts of Enteromorpha intestinalis (Chlorophyta, Ulvophyceae) as seedstock for macroagal culture. Journal of Applied Phycology 13: 103-108, 2001].

Comme les algues pluricellulaires de petites tailles ne sont pas suffisamment abondantes dans la nature pour permettre une récolte dans l'environnement et que leur petite taille rend difficile l'identification des espèces recherchées ainsi que la récolte spécifique d'une espèce donnée, on peut mettre en œuvre un procédé permettant l'obtention d'une biomasse unialgale de cellules de macroalgues de pluricellulaires de petite taille, afin de pourvoir en extraire un principe actif utilisable en cosmétique.

Ce procédé présente l'avantage d'être utilisable en bioréacteur, et de ne pas nécessiter l'utilisation d'explants axéniques et ne fait pas intervenir de phytohormones ou d'enzymes.

Dans le cadre de leurs recherches de nouveaux ingrédients actifs cosmétiques pour la prévention et/ou le traitement des signes inesthétiques générés par les agents polluants présents dans l'atmosphère sur la peau, le cuir chevelu, les cheveux ou les muqueuses, les inventeurs se sont attachés à développer une nouvelle solution technique consistant en un procédé ayant pour but d'empêcher ou de ralentir l'apparition desdits singes inesthétiques ou bien de les éliminer, mettant en œuvre des extraits glycoliques (EG) d'une biomasse unialgale de cellules de macroalgues pluricellulaires de petite taille issues de la classe des Floridéophycées.

C'est pourquoi, selon un premier aspect, l'invention a pour objet le procédé tel que défini à la revendication 1

Dans le procédé cosmétique tel que décrit ci-dessus, la formulation cosmétique à usage topique est étalée sur la surface de la peau, ou du cuir chevelu, ou des muqueuses, ou des cheveux à traiter, puis ladite surface est massée quelques instants.

L'expression "à usage topique" utilisée dans la définition de la formulation cosmétique mise en œuvre dans le procédé cosmétique objet de la présente invention, signifie que ladite formulation est mise en œuvre par application sur la peau, qu'il s'agisse d'une application directe dans le cas d'une formulation cosmétique ou d'une application indirecte lorsque la formulation cosmétique selon l'invention est imprégnée sur un support destiné à être mis en contact avec la peau (papier, lingette, textile, dispositif transdermique, etc...).

L'expression « cosmétiquement acceptable » utilisée dans la définition de la formulation cosmétique à usage topique, mise en œuvre dans le procédé cosmétique objet de la présente invention, signifie selon la directive du Conseil de la Communauté Economique Européenne N°76/768/CEE du 27 juillet 1976 modifiée par la directive N°93/35/CEE du 14

EP 3 519 056 B1

juin 1993, que ladite formulation comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Selon un aspect particulier du procédé tel que défini dessus, ledit extrait glycolique (EG) est présent dans ladite formulation cosmétique à usage topique pour 100% de sa, en une proportion comprise entre 0,1% et 3% massique et plus particulièrement entre 0,5% et 2% massique de l'extrait glycolique (EG).

Par « macroalgue pluricellulaire de petite taille », on désigne au sens de la présente invention, une macroalgue pluricellulaire mesurant entre 30 $\mu$m et 3 cm et organisée en amas cellulaires. Cette macroalgue pluricellulaire de petite taille se différencie d'une macroalgue pluricellulaire de grande taille car cette dernière mesure entre 5 cm et 20 cm et est organisée de façon tissulaire.

Dans le cadre du procédé selon l'invention, lesdites cellules de macroalgues pluricellulaires de petite taille sont issues de la classe des Floridéophycées, de la sous-classe des Nemaliophycidae, de l'ordre des Acrochaetiales, de la famille *Acrochatiaceae,* du genre *Acrochaetium* et de l'espèce *Acrochaetium moniliforme.*

Comme échantillon d'une macroalgue prélevé dans le milieu naturel, mis en œuvre à l'étape A) du procédé de préparation de l'extrait glycolique (EG) tel que défini ci-dessus, on désigne notamment un échantillon prélevé dans l'eau de mer, qu'il s'agisse d'un prélèvement d'eau de mer, d'un prélèvement à la surface de substrats solides comme les rochers, le sable, les coquilles, les sédiments ou encore des supports artificiels tels qu'une coque de bateau, un ponton ou une digue ; il peut aussi s'agir d'un prélèvement à la surface ou à l'intérieur d'autres végétaux (épiphyte ou endophyte) comme les algues ou les plantes marines, à la surface ou à l'intérieur d'animaux (épiphyte ou endophyte) comme les éponges, les cnidaires, les procordés, les échinodermes, les mollusques, les arthropodes, les annélides, ou les vertébrés marins.

L'échantillon de macroalgues prélevé dans le milieu naturel, mis en œuvre à l'étape A) du procédé de préparation de l'extrait glycolique (EG) tel que défini ci-dessus, est généralement très riche en biodiversité et contient une vaste sélection d'organismes vivants tels que des petits animaux, des protozoaires, des microalgues procaryotes, des microalgues eucaryotes et des macroalgues pluricellulaires.

Par « échantillon unialgal de cellules de macroalgues pluricellulaires », on entend dans le procédé tel que défini ci-dessus, une culture ne contenant qu'une seule espèce d'algues pluricellulaires.

Selon l'étape A) du procédé de préparation de l'extrait glycolique (EG) tel que défini ci-dessus, l'échantillon unialgal de cellules de macroalgues pluricellulaires de petite taille est obtenu en isolant les macroalgues visées des autres organismes l'algue visée. A cet effet, on peut mettre en œuvre des moyens de séparation physiques et/ou des moyens de séparation chimiques.

Comme moyen de séparation physique, il y a par exemple la séparation effectuée à l'aide d'une pipette en verre en utilisant l'extrémité du capillaire pour découper quelques cellules de macroalgues pluricellulaires de petite taille visées, en contrôlant l'opération visuellement, sous microscope ou sous loupe binoculaire. Il y a aussi la séparation par dilution successive des cellules de l'espèce visée de l'échantillon naturel.

Comme moyen de séparation chimique, il y a par exemple l'utilisation, d'antibiotiques pour éliminer des microalgues du type cyanobactéries, ou celle du dioxyde de germanium pour éliminer des microalgues du type diatomée.

Les différents moyens d'isolement sont combinés pour obtenir le meilleur résultat d'isolement possible. Toutes les méthodes d'isolement, physiques et chimiques, sont réalisées dans des réceptacles translucides laissant passer la lumière, contenant de l'eau de mer stérile, contenant au moins une source d'azote telle que le nitrate de sodium ($NaNO_3$) à une concentration comprise entre 50mg/dm$^3$ et 250mg/dm$^3$ avec une préférence pour 150mg/dm$^3$ et une source de phosphore telle que le dihydrogénophosphate de sodium ($NaH_2PO_4$) à une concentration comprise entre 5mg/dm$^3$ et 75mg/dm$^3$ avec une préférence pour 50mg/dm$^3$. Il est également possible d'ajouter à l'eau de mer d'autres éléments minéraux, par addition par exemple d'un milieu nutritif dans les proportions souhaitées comme le milieu de Provasoli de composition suivante :

| Milieu de Provasoli | | | |
|---|---|---|---|
| $NaNO_3$ | 350 mg | $ZnSO_4, 7H_2O$ | 0,55 mg |
| glycérophosphate de sodium | 50 mg | $CoSO_4, 7H_2O$ | 0,12 mg |
| $Fe(NH_4)_2(SO_4)_2, 6H_2O$ | 18 mg | Vitamine B12 | 10 $\mu$g |
| $Na_2EDTA$ | 15 mg | Thiamine | 0,5 mg |
| $H_3BO_3$ | 28,5 mg | Biotine | 5 $\mu$g |
| $FeCl_3, 6H_2O$ | 1,225 mg | Tampon Tris | 500 mg |

(suite)

| Milieu de Provasoli | | | |
| --- | --- | --- | --- |
| $MnSO_4$, $H_2O$ | 4,1 mg | eau distillée | 100 ml |

La mise œuvre de l'étape B) du procédé de préparation de l'extrait glycolique (EG) tel que défini ci-dessus est effectuée dans un photobioréacteur contenant de l'eau de mer contenant au moins une source d'azote telle que le nitrate de sodium ($NaNO_3$) à une concentration comprise entre 50mg/dm$^3$ et 250mg/dm$^3$ avec une préférence pour 150mg/dm$^3$ et une source de phosphore telle que le dihydrogénophosphate de sodium ($NaH_2PO_4$) à une concentration comprise entre 5mg/dm$^3$ et 75mg/dm$^3$ avec une préférence pour 50mg/dm$^3$.

Il est également possible d'ajouter à l'eau de mer d'autres éléments minéraux, par addition par exemple dans les proportions souhaitées du milieu de Provasoli.

Les cultures sont conduites dans des cuves de culture translucides sous bouillonnement d'air optionnellement enrichi en dioxyde de carbone.

Les cultures sont généralement conduites entre 10°C et 25°C avec une préférence pour 17°C, sous une illumination constante.

Les cultures sont conduites sur des périodes de quinze jours dans des volumes allant de 500cm$^3$ pour les premières étapes de culture jusqu'à 20m$^3$ pour les étapes de production de biomasse industrielle. Cependant, lorsque des observations à la loupe binoculaire et/ou au microscope faites à l'issue de la première période de quinze jours mettent en évidence la croissance d'autres algues que celle visée à l'issue de l'étape A) du procédé de préparation de l'extrait glycolique (EG), cela constitue le signe que le résultat de ladite étape A) n'est pas satisfaisant. Cette étape A) du procédé de préparation de l'extrait glycolique (EG) tel que défini précédemment, est donc être réitérée jusqu'à l'obtention d'un échantillon unialgal de cellules de macroalgues pluricellulaires de petite taille de qualité satisfaisante

L'étape C) du procédé de préparation de l'extrait glycolique (EG) tel que défini ci-dessus, est généralement réalisée en utilisant une toile filtrante au seuil de coupure compris entre 25$\mu$m et 100$\mu$m en fonction de la taille des macroalgues pluricellulaires de petite taille mises en culture. La culture de biomasse unialgale de cellules de macroalgues pluricellulaires de petite taille est filtrée, l'eau de mer passant à travers la toile et la biomasse restant à sa surface. Le retentât constitué par la biomasse est ensuite pressé pour éliminer l'eau libre encore présente.

Le ratio volumique eau sur glycol du mélange eau glycol mis en œuvre dans le procédé de préparation dudit extrait glycolique (EG) est plus particulièrement inférieur ou égal à 1/2 et supérieur ou égal à 1/6. Si nécessaire ou si désiré ce ratio volumique est ajusté dans l'intervalle susmentionné en fin de l'étape E) du procédé de préparation de l'extrait glycolique (EG).

Lors de l'étape F) du procédé de préparation de l'extrait glycolique (EG) tel que défini ci-dessus, la séparation des phases non miscibles est réalisée, par décantation par gravité ou par centrifugation. Si nécessaire ou si désiré l'extrait hydroglycolique obtenu est filtré à travers un filtre de seuil de coupure de 0.2 $\mu$m pour éliminer toute particule en suspension et permettre d'obtenir une solution limpide.

Par "signes inesthétiques générés par l'ozone présentes dans l'atmosphère sur la peau, le cuir chevelu, les cheveux ou les muqueuses », on entend au sens de la présente invention toute modification de l'aspect extérieur de la peau, du cuir chevelu, des cheveux ou des muqueuses due aux conséquences de l'exposition à des agents polluants atmosphériques, comme les rougeurs, les rides et ridules, l'altération du microrelief de la peau du cuir chevelu ou des muqueuse, le ternissement du teint de la peau, du cuir chevelu et des muqueuses, ou une augmentation de la porosité, du ternissement et de la fragilisation des cheveux vis-à-vis de contraintes mécaniques.

Selon un aspect plus particulier de la présente invention, celle-ci a pour objet le procédé tel que défini précédemment pour lequel l'étape E) du procédé de préparation de l'extrait glycolique (EG), tel que défini ci-dessus, se caractérise par la dispersion de ladite biomasse unialgale de cellules de macroalgues pluricellulaires de petite taille obtenue à l'étape D) d'un mélange eau-glycol mis en œuvre est un mélange eau/1,3-butanediol dont la teneur massique en 1,3-butanediol (ratio masse 1,3-butanediol sur masse totale 1,3-butanediol et eau) est comprise entre 50% et 75%, à raison de 2% massique à 10% massique de biomasse pour 100% massique de dispersion ; et pour laquelle l'agitation est maintenue pendant une à deux heures, puis si nécessaire ou si désiré, puis la teneur massique en 1,3-butanediol est ajustée à 40% par ajout d'eau.

Les formulations cosmétiques à usage topique mises en œuvre dans le procédé ayant pour but d'empêcher ou de ralentir l'apparition des signes inesthétiques générés par l'ozone présente dans l'atmosphère sur la peau, le cuir chevelu, les cheveux ou les muqueuses, ou bien d'éliminer lesdits signes, objet de la présente invention et tel que défini précédemment, se présentent généralement sous forme d'une solution aqueuse ou hydro-alcoolique ou hydroglycolique, sous forme d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile, ou sous forme d'une poudre.

Les formulations cosmétiques à usage topique mises en œuvre dans le procédé ayant pour but d'empêcher ou de ralentir l'apparition des signes inesthétiques générés par l'ozone présentes dans l'atmosphère sur la peau, le cuir chevelu, les cheveux ou les muqueuses, ou bien d'éliminer lesdits signes, objet de la présente invention et tel que défini précédemment, peuvent être conditionnées dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

De façon générale, l'extrait glycolique (EG), obtenu selon le procédé de préparation tel que défini précédemment, et présent dans les formulations cosmétiques à usage topique mises en œuvre dans le procédé objet de la présente invention, est associé à des additifs chimiques habituellement mis en œuvre dans le domaine des formulations à usage topique, comme les tensioactifs moussants et/ou détergents, les tensioactifs épaississants et/ou gélifiants, les agents épaississants et/ou gélifiants, les agents anti-pollution, les agents stabilisants, les composés filmogènes, les solvants et co-solvants, les agents hydrotropes, les eaux thermales ou minérales les agents plastifiants, les agents émulsionnants et co-émulsionnants, les agents opacifiants, les agents nacrants, les agents surgraissants, les séquestrants, les agents chélatants, les huiles, les cires, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Par «agents anti-pollution » que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les composés capables de piéger l'ozone, les composés capables de piéger les composes aromatiques mono- ou polycycliques tels que le benzopyrène, les métaux et les métaux lourds.

Parmi les agents piégeurs d'ozone que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les phénols et polyphénols, en particulier les tannins; les extraits de feuille d'olivier; les extraits de thé, en particulier de thé vert; les anthocyanes; les extraits de romarin; les acides phénoliques, en particulier l'acide chlorogénique; les stilbènes, en particulier le resveratrol; les dérivés d'acides amines soufrés, en particulier la S-carboxyméthylcystéine; l'ergothionéine; la N-acétylcystéine; des chélatants comme la N,N'-bis-(3,4,5-triméthoxybenzyl)éthylènediamine ou l'un de ses sels, complexes métalliques ou esters; des caroténoïdes tels que la crocétine; et des matières premières diverses comme le mélange d'arginine, de ribonucléate, d'histidine, de mannitol, d'adénosinetriphosphate, de pyridoxine, de phénylalanine, de tyrosine et d'ARN hydrolysé commercialisé par les Laboratoires Sérobiologiques sous la dénomination commerciale CPP LS 2633-12F™, la fraction hydrosoluble de maïs commercialisée par la société SOLABIA sous la dénomination commerciale Phytovityl™, le mélange d'extrait de fumeterre et d'extrait de citron commercialisé sous la dénomination Unicotrozon C-490 par la société Induchem, et le mélange d'extraits de ginseng, de pomme, de pèche, de blé et d'orge vendu par la société PROVITAL sous la dénomination commerciale Pronalen Bioprotect™.

Parmi les agents piégeurs de composés aromatiques mono- ou polycycliques que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les dérivés d'indoles, en particulier l'indol-3-carbinol; les extraits de thé en particulier de thé vert, les extraits de Jacinthe d'eau ou Eichornia crassipes; et la fraction hydrosoluble de maïs commercialisée par la société SOLABIA sous la dénomination commerciale Phytovityl™.

Parmi les agents piégeurs de métaux lourds que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters; l'acide phytique; les dérivés de chitosan; les extraits de thé, en particulier de thé vert; les extraits de Jacinthe d'eau (Eichornia crassipes); et la fraction hydrosoluble de maïs commercialisée par la société SOLABIA sous la dénomination commerciale Phytovityl™.

Parmi les solvants, et plus particulièrement parmi les solvants volatils que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les alcools hydrosolubles et volatils, comme l'éthanol, l'isopropanol ou le butanol, et plus particulièrement l'éthanol, les solvants organiques comme le glycérol, le diglycérol, les oligomères du glycérol, le propane-1,2 diol, le propane-1,3 diol, le butane-1,3 diol, le butane-1,4 diol, l'hexane-1,6 diol, le 2-méthylpentane-2,4-diol, le pentane-1,2 diol, le 2-methyl-1,3-propanediol, le 2-Méthylpentane-2,4-diol (ou hexylène glycol), le dipropylene glycol, le xylitol, l'érythritol, le sorbitol.

Parmi les tensioactifs moussants et/ou détergents que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les tensioactifs moussants et/ou détergents anioniques, cationiques, amphotères ou non ioniques.

Parmi les tensioactifs anioniques moussants et/ou détergents que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les sels de métaux alcalins, les sels de métaux alcalino-terreux, les sels d'ammonium, les sels d'amines, les sels d'amino alcools d'alkyléthers sulfates, d'alkyl sulfates, d'alkylamidoéther sulfates, d'alkylarylpolyéther sulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkylsulfosuccinates, d'alkyléther sulfosuccinates, d'alkylamide sulfosuccinates, d'alkyl sulfo-acétates, d'alkyl sarcosinates, d'acyliséthionates, de N-acyl taurates, d'acyl lactylates, de dérivés N-acylés d'acides aminés, de dérivés N-acylés de peptides, de dérivés N-acylés de protéines, d'acides gras.

Parmi les tensioactifs amphotères moussants et/ou détergents que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer particulièrement les dérivés d'ammoniums quaternaires.

Parmi les tensioactifs non ioniques moussants et/ou détergents que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer plus particulièrement les alkylpolyglycosides comportant un radical aliphatique, linéaire ou ramifié, saturé ou insaturé, et comportant de 8 à 12 atomes de carbone comme par exemple l'octylpolyglucoside, le décylpolyglucoside, le laurylpolyglucoside, le cocoylpolyglucoside, ou leurs mélanges ; les dérivés d'huile de ricin, les polysorbates, les amides de coprah, les N-alkylamines.

Comme exemples d'agents de texture que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer des dérivés N-acylés d'acides aminés, par exemple la lauroyl lysine commercialisée sous l'appellation AMINOHOPE™LL, l'octenyl starch succinate commercialisé sous l'appellation DRYFLO™, le myristyl polyglucoside commercialisé sous l'appellation MONTANOV™ 14, les fibres de cellulose, les fibres de coton, les fibres de chitosane, le talc, la séricite, le mica, la perlite.

Comme exemples de principes actifs que l'on peut associer à l'extrait glycolique (EG), obtenu selon le procédé de préparation tel que défini précédemment, et présent dans les formulations cosmétiques à usage topique mises en oeuvre dans le procédé objet de la présente invention, on peut citer :

- Les vitamines et leurs dérives, par exemple, le rétinol (vitamine A) et ses esters (palmitate de rétinyle par exemple), l'acide ascorbique (vitamine C) sous forme de sel et ses esters, les dérives de sucre de l'acide ascorbique (par exemple l'ascorbyl glucoside), le tocophérol (vitamine E) et ses esters (par exemple l'acétate de tocophérol), les vitamines B3 ou B10 (niacinamide et ses dérivés) ;
- Les composés ayant une action éclaircissante ou dépigmentante de la peau, par exemple le SEPIWHITE™MSH, l'arbutine, l'acide kojique, l'hydroquinone, le VEGEWHITE™, la GATULINE™, le SYNERLIGHT™, le BIOWHITE™, le PHYTOLIGHT™, le DERMALIGHT™, la CLARISKIN™, le MELASLOW™, le DERMAWHITE™, l'ETHIOLINE, le MELAREST™, le GIGAWHITE™, l'ALBATINE™, le LUMISKIN™, le SEASHINE™, le Celtosome™ *Crithmum maritimum* ;
- Les composés ayant une action apaisante comme le SEPICALM™ S, l'allantoïne et le bisabolol ;
- Les agents anti-inflammatoires ;
- Les composés montrant une action hydratante par exemple le diglycérol, le triglycérol, l'urée, les hydroxyurées, le glycérolglucoside, le diglycérolglucoside, les polyglycérylglucosides, l'érythritylglucoside, le sorbitylglucoside, le xylitylglucoside, la composition commercialisée sous le nom de marque AQUAXYL™ comprenant du xylitylglucoside, de l'anhydroxylitol et du xyllitol ; les extraits d'algues commercialisés sous les noms de marque CODIAVELANE™ et BIOPLASMA™.
- Les composés montrant une action amincissante ou lipolytique comme la caféine ou ses dérivés, l'ADIPOSLIM™, l'ADIPOLESS™, le RODYSTEROL™ ;
- Les protéines N-acylées ; les peptides N-acylés par exemple le MATRIXIL™ ; les acides aminés N-acylés ; les hydrolysâts partiels de protéines N-acylés ; les acides aminés ; les peptides ; les hydrolysâts totaux de protéines ;
- Les extraits végétaux riches en tanins en polyphénols et/ ou en isoflavones, par exemple les extraits de raisin, les extraits de pin, les extraits de vin, les extraits d'olives ; les extraits de soja, par exemple la Raffermine™ ; les extraits de blé par exemple la TENSINE™ ou la GLIADINE™ ; les extraits végétaux riches en terpènes ; les extraits d'algues d'eau douce ou marines ; les extraits marins en général comme les coraux ;

- Les composés ayant une action antimicrobienne ou une action purifiante, par exemple le LIPACIDE™ C8G, le LIPACIDE™ UG, le SEPICONTROL™ A5 ; l'OCTOPIROX™ ou le SENSIVA™ SC50, la PHLOROGINE™ ;
- Les composés ayant une propriété énergisante ou stimulante comme le Physiogényl™, le panthénol et ses dérivés comme le SEPICAP™ MP, le SEPITONIC™M3, le SEPITONIC™M4 ;
- Les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™, la KALPARIANE™, l'ANTILEUKINE-6™, l'EPHEMER™, la JUVENESSENCE™, les CELTOSOMES™ *Erygium maritimum,* le CERAMOSIDE™ ;
- Les actifs anti-photo vieillissement ;
- Les actifs augmentant la synthèse des composants de la matrice extracellulaire par exemple le collagène, les élastines, les glycosaminoglycanes ;
- Les actifs agissant favorablement sur la communication cellulaire chimique comme les cytokines ou physiques comme les intégrines ;
- Les actifs créant une sensation de « chauffe » sur la peau comme les activateurs de la microcirculation cutanée (par exemple les dérivés de l'acide nicotinique) ou des produits créant une sensation de « fraîcheur » sur la peau (par exemple le menthol et des dérivés) ;
- Les actifs améliorant la microcirculation cutanée, par exemple les veinotoniques ; les actifs drainants ; les actifs à visée décongestionnante par exemple les extraits de ginko biloba, de lierre, de marron d'inde, de bambou, de ruscus, de petit houx, de *centalla asiatica,* de fucus, de romarin, de saule ;
- les actifs agissant comme agents tenseurs de la peau, par exemple les hydrolysats de protéines végétales, les hydrolysats d'origine marine comme les hydrolysats d'extraits de laminaires, les hydrolysats de cartilages de poissons, l'élastine marine, le produit commercialisé par la société SEPPIC sous le nom de marque SESAFLASH™, les solutions de collagène.
- Les agents de bronzage ou de brunissement de la peau, par exemple la dihydroxyacétone, l'isatine, l'alloxane, la ninhydrine, le glycéraldéhyde, l'aldéhyde mésotartrique, la glutaraldéhyde, l'érythrulose, les extraits végétaux par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683 ; les agents connus pour leur action de facilitation et/ou d'accélération du bronzage et/ou du brunissement de la peau humaine, et/ou pour leur action de coloration de la peau humaine, par exemple les caroténoïdes ( et plus particulièrement le beta carotène et le gamma carotène), le produit commercialisé sous le nom de marque « Carros oil » (Nom INCI : Daucus Carotta, hélianthes anus Sunflower oil) par la société Provital, qui contient des caroténoïdes, de la vitamine E et de la vitamine K ; la tyrosine et/ou ses dérivés, connus pour leur effet sur l'accélération du bronzage de la peau humaine en association avec une exposition aux rayonnements ultra-violets, par exemple le produit commercialisé sous le nom de marque « SunTan Accelerator™ » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze™ (nom INCI : Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI : butylene glycol and Acetyl Tyrosine and hydrolyzed vegetable protein and Adenosine triphosphate) par la société UNIPEX, le produit commercialisé sous le nom de marque « Try-Excell™ » (nom INCI : Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze™ » (nom INCI : hydrolyzed wheat protein and acetyl tyrosine and copper gluconate) par la société Alban Muller, le produit commercialisé sous le nom de marque Tyrostan™ (nom INCI : potassium caproyl tyrosine) par la société Synerga, le produit commercialisé sous le nom de marque Tyrosinol (nom INCI : Sorbitan Isostearate, glyceryl oleate, caproyl Tyrosine) par la société Synerga, le produit commercialisé sous le nom de marque InstaBronze™ (nom INCI : Dihydroxyacetone and acetyl tyrosine and copper gluconate) commercialisé par la société Alban Muller, le produit commercialisé sous le nom de marque Tyrosilane (nom INCI : méthylsilanol and acétyl tyrosine) par la société Exymol ; les peptides connus pour leur effet d'activation de la mélanogénèse par exemple le produit commercialisé sous le nom de marque Bronzing SF Peptide powder (nom INCI : Dextran and Octapeptide-5) par la société Infinitec Activos, le produit commercialisé sous le nom de marque Melitane (nom INCI : Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions™ (nom INCI : Butylene glycol , Palmitoyl Tripeptide-40) par la société LIPOTEC, les sucres et les dérivés de sucres par exemple le produit commercialisé sous le nom de marque Tanositol™ (nom INCI : inositol) par la société Provital, le produit commercialisé sous le nom de marque Thalitan™ (ou Phycosaccharide™ AG) par la société CODIF international (nom INCI : Aqua and hydrolyzed algin (Laminaria Digitata) and magnesium sulfate and manganese sulfate) contenant un oligosaccharide d'origine marine (acide guluronique et acide mannuronique chélatés avec les ions magnésium et manganèse), le produit commercialisé sous le nom de marque Melactiva™ (nom INCI : Maltodextrin, Mucuna Pruriens Seed extract) par la société Alban Muller, les composés riches en flavonoïdes par exemple le produit commercialisé sous le nom de marque « Biotanning » (nom INCI : Hydrolyzed citrus Aurantium dulcis fruit extract) par la société Silab et connu pour être riche en flavonoides de citron (de type hespéridines) ; les agents destinés au traitement des cheveux et/ou des poils, par exemple des agents protecteurs des mélanocytes du follicule pileux, destinés à protéger lesdits mélanocytes contre les agents cytotoxiques responsables de la sénescence et/ou de l'apoptose desdits mélanocytes, tels que les agents mimétiques de l'activité de la DOPAchrome tautomérase choisis parmi ceux décrits dans la demande de brevet européen publiée sous le numéro EP 1 515 688 A2, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, des associations de mono- et diester d'acide cinnamique et de vitamine C, et plus généralement ceux cités dans la demande de brevet européen publiée sous le numéro EP 1 515 688 A2.

Comme exemples d'agents déodorants que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2-décanediol ; le 1,3-propanediol ; l'acide salicylique ; le bicarbonate de sodium ; les cyclodextrines ; les zéolithes métalliques ; le Triclosan™ ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples de filtres solaires que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer tous ceux figurant dans la directive cosmétique 76/768/CEE modifiée annexe VII.

Parmi les filtres organiques solaires que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ; la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxy éthyle, le cinnamate de p-méthoxy cyclohexyle, le cinnamate d'éthyl-α-cyano-β-phényle, le cinnamate de 2-éthylhexyl-α-cyano-β-phényle, le cinnamate de diparaméthoxy mono-2-éthylhexanoyl de glycéryle ; la famille des dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-carboxylate, la 2-hydroxy-4-n-octyloxybenzophénone, la 4-hydroxy-3-carboxybenzophénone ; le 3-(4'-méthylbenzylidène)-d,l-camphre, le 3-(benzylidène)-d,l-camphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ; la famille des dérivés de la triazine comme l'hydroxyphényl triazine, l'éthylhexyloxyhydroxyphényl-4-méthoxyphényltriazine, le 2,4,6-trianilino-(p-carbo-2'-éthylhexyl-1'-oxy)-1,3,5-triazine, le 4,4-((6-(((1,1-diméthyléthyl) amino) carbonyl) phenyl) amino)-1,3,5-triazine-2,4-diyl diimino) bis-(2-éthylhexyl) ester de l'acide benzoïque, le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-t-butylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate ; la famille des polysiloxanes comme le malonate de benzylidène siloxane ; plus particulièrement le 2-(4-diethylamino-2-hydroxybenzoyl)benzoic acid hexyl ester, le 2,4-bis{[4-(2-ethylhexyloxy)-2-hydroxy]phenyl}-6-(4-methoxyphenyl)-1,3,5-triazine, le 2,4,6-tris[4-(2 ethylhexyloxycarbonyl)anilino]-1,3,5-triazine et le 2-ethylhexyl dimethoxybenzylidene dioxoimidazolidine propionate.

Parmi les filtres inorganiques solaires, également appelés "écrans minéraux", que l'on peut associer à l'extrait glycolique (EG), obtenu selon le procédé de préparation tel que défini précédemment, et présent dans les formulations cosmétiques à usage topique mises en oeuvre dans le procédé objet de la présente invention, on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

Comme exemples de tensioactifs épaississants et/ou gélifiants que l'on peut associer à l'extrait glycolique (EG), obtenu selon le procédé de préparation tel que défini précédemment, et présent dans les formulations cosmétiques à usage topique mises en oeuvre dans le procédé objet de la présente invention, on peut citer les esters gras d'alkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ; les esters gras alcoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141 ; les carbamates de polyalkylène glycols à chaînes grasses comme le PPG-14 laureth isophoryl dicarbamate commercialisé sous l'appellation ELFACOS™ T211, le PPG-14 palmeth-60 hexyl dicarbamate commercialisé sous l'appellation ELFACOS™ GT2125.

Comme exemples d'agents épaississants et/ou gélifiants que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide 2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- methyl)acrylamido methane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymère linéaire, branché ou réticulé d'au moins un monomère possédant une fonction acide fort, libre, partiellement salifiée ou totalement salifiée, avec au moins un monomère neutre, et au moins un monomère de formule (I) :

$$CH_2=C(R'_3)-C(=O)-[CH_2-CH_2-O]_n-R'_4 \qquad (I)$$

dans laquelle $R'_3$ représente un atome d'hydrogène ou un radical méthyle, $R'_4$ représente un radical alkyle linéaire ou ramifié comportant de huit à trente atomes de carbone et n représente un nombre supérieur ou égal à un et inférieur ou égal à cinquante.

Les polymères de type polyélectrolytes, linéaires ou branchés ou réticulés que l'on peut associer à l'extrait glycolique (EG), obtenu selon le procédé de préparation tel que défini précédemment, et présent dans les formulations cosmétiques à usage topique mises en œuvre dans le procédé objet de la présente invention, peuvent se présenter sous la forme d'une solution, d'une suspension aqueuse, d'une émulsion eau-dans-huile, d'une émulsion huile-dans-eau, d'une poudre. Les polymères de type polyélectrolytes, linéaires ou branchés ou réticulés que l'on peut associer à l'extrait glycolique (EG), obtenu selon le procédé de préparation tel que défini précédemment, et présent dans les formulations cosmétiques à usage topique mises en œuvre dans le procédé objet de la présente invention, peuvent être sélectionnés parmi les produits commercialisés sous les appellations SIMULGEL™ EG, SIMULGEL™EPG, SEPIGEL™ 305, SIMUL-

GEL™ 600, SIMULGEL™ NS, SIMULGEL™ INS 100, SIMULGEL™ FL, SIMULGEL™ A, SIMULGEL™ SMS 88, SEPINOV™EMT 10, SEPIPLUS™400, SEPIPLUS™265, SEPIPLUS™S, SEPIMAX™Zen, ARISTOFLEX™AVC, ARISTOFLEX™AVS, NOVEMER™EC-1, NOVEMER™EC 2, ARISTOFLEX™HMB, COSMEDIA™SP, FLOCARE™ET 25, FLOCARE™ET 75, FLOCARE™ET 26, FLOCARE™ET 30, FLOCARE™ET 58, FLOCARE™PSD 30, VISCOLAM™AT 64, VISCOLAM™AT 100.

Comme exemples d'agents épaississants et/ou gélifiants que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ).

Comme exemples d'agents épaississants et/ou gélifiants que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Comme exemples d'agents épaississants et/ou gélifiants que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples d'eaux thermales ou minérales que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les xylènes sulfonates, les cumènes sulfonates, le n-hexyl polyglucoside, le (2-éthyl hexyl) polyglucoside ou le n-heptyl polyglucoside.

Comme exemples d'agents tensioactifs émulsionnants que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les tensioactifs non ioniques, des tensioactifs anioniques, des tensioactifs cationiques.

Comme exemples de tensioactifs non-ioniques émulsionnants que l'on peut associer à l'extrait glycolique (EG), obtenu selon le procédé de préparation tel que défini précédemment, et présent dans les formulations cosmétiques à usage topique mises en oeuvre dans le procédé objet de la présente invention, on peut citer les esters d'acides gras et de sorbitol, comme les produits commercialisés sous les appellations MONTANE™40, MONTANE™60, MONTANE™70, MONTANE™80 et MONTANE™85 ; les compositions comprenant du stéarate de glycérol et l'acide stéarique éthoxylé entre cinq moles et cent cinquante moles d'oxyde d'éthylène, comme la composition comprenant de l'acide stéarique éthoxylé à cent trente-cinq moles d'oxyde d'éthylène et du stéarate de glycérol commercialisée sous l'appellation SIMULSOL™ 165 ; les esters de mannitan ; les esters de mannitan éthoxylés ; les esters de sucrose ; les esters de méthyl glucoside ; les alkyl polyglycosides comportant un radical aliphatique, linéaire ou ramifié, saturé ou insaturé, et comportant de quatorze à trente-six atomes de carbone, comme le tétradécyl polyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le (2-octyl dodécyl) polyxyloside, le (12-hydroxy stéaryl) polyglucoside ; les compositions d'alcools gras linéaires ou ramifiés, saturés ou insaturés, et comportant de quatorze à trente-six atomes de carbone, et d'alkyl polyglycosides tels que décrits précédemment, par exemple les compositions commercialisées sous les noms de marque MONTANOV™68, MONTANOV™14, MONTANOV™82, MONTANOV™202, MONTANOV™S, MONTANOV™WO18, MONTANOV™L, FLUIDANOV™20X et EASYNOV™.

Comme exemples de tensioactifs anioniques que l'on peut associer à l'extrait glycolique (EG), obtenu selon le procédé de préparation tel que défini précédemment, et présent dans les formulations cosmétiques à usage topique mises en oeuvre dans le procédé objet de la présente invention, on peut citer le glycéryl stéarate citrate, le cétéarylsulfate, les savons comme le stéarate de sodium ou le stéarate de triéthanolammonium, les dérivés N-acylés d'acides aminés salifiés comme par exemple le stéaroyl glutamate.

Comme exemples de tensioactifs cationiques émulsionnants que l'on peut associer à l'extrait glycolique (EG), obtenu selon le procédé de préparation tel que défini précédemment, et présent dans les formulations cosmétiques à usage topique mises en oeuvre dans le procédé objet de la présente invention, on peut citer les aminoxydes, le quaternium-82 et les tensioactifs décrits dans la demande de brevet WO 96/00719 et principalement ceux dont la chaîne grasse comprend au moins seize atomes de carbone.

Comme exemples d'agents opacifiants et/ou nacrants que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de douze à vingt-deux atomes de carbone.

Comme exemples d'huiles que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer les huiles minérales telles que l'huile de paraffine, l'huile de vaseline, les isoparaffines ou les huiles blanches minérales ; les huiles d'origine animale, telles que le squalène ou le squalane ;les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de graines de coriandre, l'huile de faine, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires que l'on peut associer à l'extrait glycolique (EG), obtenu selon le procédé de préparation tel que défini précédemment, et présent dans les formulations cosmétiques à usage topique mises en œuvre dans le procédé objet de la présente invention, on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

L'invention a aussi pour objet un extrait glycolique (EG) d'une biomasse unialgale de cellules de macroalgues pluricellulaires de petite taille issues de la classe des Floridéophycées et obtenu par le procédé comprenant les étapes successives suivantes :

- Une étape A) de préparation d'un échantillon unialgal de cellules de macroalgues pluricellulaires, à partir d'un échantillon de macroalgues issues de l'espèce Acrochaetium moniliforme et prélevé dans le milieu ;

  - Une étape B) de mise en culture dudit échantillon unialgal de cellules de macroalgues pluricellulaires obtenu à l'étape A) dans l'eau de mer additionnée de nitrate de sodium (NaNO3) à une concentration comprise entre 50 mg/dm3 et 250 mg/dm3 et de dihydrogénophosphate de sodium (NaH2PO4) à une concentration comprise entre 5 mg/dm3 et 75 mg/dm3 et à une température comprise entre 10°C et 25°C, pour obtenir une suspension aqueuse de ladite biomasse unialgale de cellules de macroalgues pluricellulaires de petite taille, ladite étape B étant effectuée dans un photobioréacteur;

- Une étape C) de récolte de ladite biomasse unialgale de cellules de macroalgues pluricellulaires de petite taille à partir de ladite suspension aqueuse obtenue à l'issue de l'étape B) au moyen d'une toile filante au seuil de coupure compris entre 25$\mu$m et 100$\mu$m puis pressage de la biomasse obtenue pour éliminer l'eau résiduelle ;

- une étape D) de préparation d'une poudre de ladite biomasse unialgale de cellules de macro-algues pluricellulaires de petite taille obtenue à l'étape C), par congélation de ladite biomasse puis sa lyophilisation son broyage ;

- Une étape E) au cours de laquelle la poudre de ladite biomasse à l'étape D), est dispersée sous agitation dans un mélange eau-1,3-butanediol dans lequel le ratio volumique eau sur 1,3-butanediol est inférieur ou égal à 1/1 et supérieur ou égal à 1/9, pour former une dispersion de ladite biomasse dans ledit mélange eau-1,3-butanediol comprenant pour 100% de sa masse, de 1% massique à 20% massique deladite biomasse; et

- Une étape F) au cours de laquelle la dispersion obtenue à l'étape E) précédente, est séparée en ses phases non miscibles, pour recueillir ledit extrait glycolique (EG).

Les exemples suivants illustrent l'invention sans toutefois la limiter.

**1- Evaluation de l'effet d'un extrait glycolique (dans le 1,3 butanediol) d'une biomasse unialgale de cellules de macroalgues pluricellulaires de petite taille issues de l'algue *Acrochaetium moniliforme*, nommé « Extrait A », sur une culture de kératynocytes en présence d'un agent polluant atmosphérique (le cadmium).**

L'extrait A est obtenu par la mise en oeuvre du procédé de préparation de l'extrait glycolique (EG) tel que décrit précédemment, pour lequel :

- dans l'Etape A) du procédé de préparation de l'extrait glycolique (EG), telle que décrite précédemment, la macroalgue rouge, prélevée dans le milieu naturel, à savoir dans l'eau de mer de la Manche sur le rivage des Côtes d'Armor, est l'algue *Acrochaetium moniliforme,*

- dans l'Etape E) du procédé de préparation de l'extrait glycolique (EG), le glycol utilisé est le 1,3 Butanediol, à raison de 40 % massique pour 100% de la masse de la dispersion,

Principe de l'évaluation : évaluation des effets d'une exposition au cadmium sur une culture de keratinocyte.

Protocole : Des kératinocytes humains sont cultivés en plaque de culture 96 puits. Après quelques jours de culture, les produits évalués, à savoir l'Extrait A à 0,5% massique et le sulforaphane (une référence positive connue de l'homme du métier), sont appliqués dans le milieu de culture. A l'issue d'une incubation de 24 heures, les cellules sont exposées à une solution de cadmium à 35$\mu$M pendant 24 heures, puis 24 heures après, les différentes évaluations suivantes sont menées :

- évaluation de la viabilité cellulaire à l'aide d'un dosage ADN par la méthode Hoechst évaluation du métabolisme cellulaire à l'aide d'un dosage de protéines par la méthode BCA ;

- évaluation de la composante inflammatoire à l'aide d'un dosage des cytokines IL-1$\alpha$ et IL-8.

Résultats : Les résultats relatifs à la viabilité cellulaire des produits évalués sont consignés dans le tableau 1 suivant :

**Tableau 1**: évaluation de la viabilité cellulaire de l'Extrait A) selon l'invention.

| Produits testés | Valeurs moyennes des protéines exprimées ($\mu$g/ml) | Ecart-type associé ($\mu$g/ml) |
|---|---|---|
| Cellules témoins (non polluées, non traitées) | 300,7 | 5,56 |
| Cellules polluées et non traitées | 65,2 | 5,33 |
| Cellules polluées associées au sulforaphane à 1$\mu$M | 156, 7 | 25,25 |
| Cellules polluées associées avec l'extrait A) à 0,5% | 233,2 | 15,6 |

Les résultats relatifs à l'évaluation du métabolisme cellulaire des produits évalués sont consignés dans le tableau 2 suivant :

**Tableau 2**: évaluation du métabolisme cellulaire de l'extrait A) selon l'invention.

| Produits testés | Valeurs moyennes de l'ADN exprimé ($\mu$g/ml) | Ecart-type associé ($\mu$g/ml) |
|---|---|---|
| Cellules témoins (non polluées, non traitées) | 25,2 | 1,52 |
| Cellules polluées et non traitées | 2,54 | 0,44 |
| Cellules polluées associées au sulforaphane à 1$\mu$M | 6, 7 | 1,75 |
| Cellules polluées associées avec l'Extrait A) à 0,5% | 10,25 | 1,71 |

Les résultats relatifs à l'évaluation des réponses inflammatoires au regard des cytokines IL-1$\alpha$ et IL-8 des produits évalués sont consignés dans le tableau 3 suivant :

**Tableau** 3 : évaluation des réponses inflammatoires au regard des cytokines IL-1$\alpha$ de l'extrait A) selon l'invention.

| Produits testés | Valeurs moyennes d'IL-1$\alpha$ exprimées pg/$\mu$g de protéines) | Ecart type associé (pg/$\mu$g de protéines) |
|---|---|---|
| Cellules témoins (non polluées, non traitées) | 0,2735 | 0,0389 |
| Cellules polluées et non traitées | 6,7843 | 0,8304 |
| Cellules polluées associées au Sulforaphane à 1$\mu$M | 0,8238 | 0,3765 |
| Cellules polluées associées avec l'extrait A) à 0,5% | 0,2831 | 0,0995 |

**Tableau 3 (suite)** : Evaluation des réponses inflammatoires au regard des cytokines IL-8 de l'extrait A) selon l'invention.

| Produits testés | Valeurs moyennes d'IL-8 exprimées (pg/$\mu$gde protéines) | Ecart type associé (pg/$\mu$g de protéines) |
|---|---|---|
| Cellules témoins (non polluées, non traitées) | 0,4499 | 0,1235 |
| Cellules polluées et non traitées | 6,6947 | 0,9727 |
| Cellules polluées associées au Sulforaphane à 1$\mu$M | 3,7081 | 0,6135 |
| Cellules polluées associées avec l'extrait A) à 0,5% | 2,5154 | 0,1850 |

L'application de cadmium dans le milieu de culture entraîne une diminution de la valeur moyenne des protéines exprimées de 300,7$\mu$g/ml à 65,2 $\mu$g/ml (voir Tableau 1), soit une diminution de 78,2%, alors que l'application de la même quantité de cadmium dans le milieu de culture comprenant 0,5% massique de l'Extrait A) se traduit par une valeur moyenne de protéine exprimée de 233,2$\mu$g/ml (voir Tableau 1), soit une diminution de seulement 22,4%. Cet effet protecteur est statistiquement significatif, selon le test de Student (avec $p<0,001$) par rapport à la condition contrôle (« Cellules polluées par Cadmium et non traitées »).

L'application de cadmium dans le milieu de culture entraîne une diminution de la valeur moyenne de la quantité d'ADN produite par les cellules de 25,12$\mu$g/ml à 2,54$\mu$g/ml (voir Tableau 2), soit une diminution de 90,3%, alors que l'application de la même quantité de cadmium dans le milieu de culture comprenant 0,5% massique de l'Extrait A) se traduit par une valeur moyenne de l'ADN produite de 10,25$\mu$g/ml (voir Tableau 2), soit une diminution limitée à 58,9%. Cet effet protecteur est statistiquement significatif, selon le test de Student (avec $p<0,01$) par rapport à la condition contrôle (« Cellules polluées par Cadmium et non traitées »).

L'application de cadmium dans le milieu de culture entraîne une augmentation de la valeur moyenne d'interleukines IL-8 produites de 0,4499pg/ $\mu$g (picogramme/microgramme) à 6,6947pg/ $\mu$g (voir Tableau 3), soit une augmentation de 1388%, alors que l'application de la même quantité de cadmium dans le milieu de culture comprenant 0,5% massique de l'Extrait A) se traduit par une valeur moyenne d'interleukines IL-8 produites de 2,5154pg/$\mu$g (voir Tableau 3), soit une augmentation de 459%. Cet effet protecteur est statistiquement significatif, selon le test de Student (avec $p<0,01$) par rapport à la condition contrôle (« Cellules polluées par Cadmium et non traitées »).

L'application de cadmium dans le milieu de culture entraîne une augmentation de la valeur moyenne d'inter-

leukines IL-1$\alpha$ produites de 0,2735pg/$\mu$g à 6,7843pg/$\mu$g (voir Tableau 3), soit une augmentation de 2380%, alors que l'application de la même quantité de cadmium dans le milieu de culture comprenant 0,5% massique de l'Extrait A) se traduit par une valeur moyenne d'interleukines IL-1$\alpha$ produites de 0,2831, soit une augmentation de 3,5%. Cet effet protecteur est statistiquement significatif, selon le test de Student (avec $p < 0,001$) par rapport à la condition contrôle (« Cellules polluées par Cadmium et non traitées »).

On peut donc conclure de ces observations expérimentales, que l'Extrait A), mis en oeuvre dans le procédé selon l'invention, se caractérise par un effet protecteur des cellules de la peau humaine soumise à l'exposition d'un agent polluant, en protégeant leur métabolisme, leur intégrité (viabilité) et en limitant les phénomènes d'inflammation, et donc ses conséquences sur la peau, les muqueuses, les cheveux et le cuir chevelu.

**2- Evaluation de l'effet d'un extrait glycolique (dans le 1,3-butanediol) d'une biomasse unialgale de cellules de macroalgues pluricellulaires de petite taille issues de l'algue *Acrochaetium moniliforme,* nommé « Extrait A », sur des patients soumis à une atmosphère polluée riche en ozone.**

*2.1 Principe de l'évaluation :*

Le squalène est un lipide propre au sébum humain qui, du fait de sa structure chimique et notamment de la présence de liaisons éthyléniques, joue un rôle de barrière anti-oxydante à la surface de la peau mais est très sensible aux phénomènes d'oxydation, et plus particulièrement à ceux impliquant l'ozone comme réactif oxydant. Il est ainsi connu dans la littérature (A. Whisthaler and C.J. Weschler, PNAS, April 13, 2010 ; Vol. 107, no.15, pp 6568-6575, « Reaction of ozone with human skin lipids : sources of carbonyls, dicarbonyls, and hydroxycarbonyls in indoor air »), que l'ozonolyse du squalène a pour conséquence la génération de nombreux produits de dégradation, dont les 3 principaux sont le C27-pentaènal, le C22-tetraènal et le C17-triènal, lesquels, à l'issue d'un processus d'ozonolyse dont ils sont eux-mêmes l'objet, génèrent des produits volatils comme par exemple l'acétone, le 6-méthyl-5-heptène-2-one, le 2,6-diméthyl-2,6-undécandièn-10-one (géranyl acétone), le 1-hydroxypropan-2-one (hydroxy acétone) ; lesdits produits volatils, notamment les espèces dicarbonylés, sont des irritants respiratoires et des irritants de la peau humaine (A. Whisthaler and C.J. Weschler, PNAS, April 13, 2010 ; Vol. 107, no.15, pp 6568-6575, « Reaction of ozone with human skin lipids : sources of carbonyls, dicarbonyls, and hydroxycarbonyls in indoor air »).

Le principe de l'évaluation de l'extrait A sur des patients soumis à une atmosphère polluée riche en ozone, réside donc à mesurer l'évolution au cours du temps de la teneur cumulée en C27-pentaènal, en C22-tetraènal et en C17-triènal présents dans le sébum des patients soumis à une atmosphère « polluée » caractérisée par sa teneur en ozone supérieure à la moyenne.

*2.2 Protocole :*

L'étude a été menée à Shanghaï à l'automne 2015, dans des conditions de pollution avérées et mesurées selon le standard international AQI (Air Quality Index) de «moderate level » à « unhealthy ».En terme de teneur en ozone, les valeurs ont été mesurées entre 6$\mu$g/m$^3$ et 183$\mu$g/m$^3$. Un groupe de 20 volontaires a été recruté pour mener à bien cette étude.

Caractéristiques du panel : Femmes chinoises, âgées de 25 à 60ans (et avant les signes de la ménopause), présentant une peau grasse avérée, avec critère d'inclusion à 140$\mu$g/cm$^2$ au niveau du front mesuré au Sébumètre® (commercialisée par la société Courage & Khazaka) en début d'étude, et/ou présentant une peau avec des imperfections et des pores dilatés.

L'étude a duré deux mois, et a consisté à préparer une émulsion huile-dans-eau dont la composition et la teneur massique de chacun des ingrédients, pour 100% de sa masse, sont consignés dans le Tableau 4 ci-dessous :

**Tableau 4 :** émulsion huile-dans-eau testée.

| Ingrédients | Teneur massique (%) |
|---|---|
| Eau | QSP 100 |
| Glycérine | 3 |
| Solagum AX[(1)] | 0.3 |
| Montanov 202[(2)] | 2 |
| Lanol 99[(3)] | 7 |
| Cetiol OE[(4)] | 3 |
| Lanol P[(5)] | 0.25 |

(suite)

| Ingrédients | Teneur massique (%) |
|---|---|
| Sepiplus 400[6] | 0.8 |
| Euxyl PE9010[7] | 1 |
| Euxyl K900[8] | 0.5 |
| Extrait A | 1 |

(1) : Solagum™ AX (nom INCI : Acacia Senegal gum & xanthan gum) est un agent émulsionnant ;
(2) Montanov™202 (nom INCI : Arachidyl alcohol & behenyl alcohol & arachidyl glucoside) est un agent émulsionnant ;
(3) Lanol™99 (nom INCI : Isononyl isononanoate) est un ester émollient ;
(4) Cetiol™ OE (nom INCI : Dicaprylyl ether) est une phase grasse cosmétique
(5) : Lanol™ P (nom INCI : Glycol palmitate) est une phase grasse cosmétique.
(6) : Sepiplus™400 (nom INCI : Polyacrylate 13 & polysorbate 20 & polyisobutene & sorbitan isostearate) est un agent épaississant, émulsionnant et stabilisant, se présentant sous la forme d'un latex inverse ;
(7) : Euxyl™ PE9010 (nom INCI : Phenoxyethanol & ethylhexylglycerin) est un agent conservateur ;
(8) : Euxyl™ K900 (nom INCI : Benzyl Alcohol &Ethylhexylglycerin & Tocopherol) est un agent conservateur.

Ladite émulsion a été administrée au groupe de 20 personnes tel que décrit précédemment pendant 56 jours, en application biquotidienne. Des prélèvements de sébum ont été effectués au niveau du front des volontaires à J0 (début d'étude) et J56 (fin d'étude).L'analyse desdits prélèvements a consisté en la recherche et le dosage des 3 composés initialement issus de l'ozonolyse du squalène, à savoir le C27-pentaènal, le C22-tetraènal et le C17-triènal, par la mise en oeuvre d'une méthode analytique impliquant une chromatographie gazeuse couplée spectrométrie de masse ; la teneur cumulée de ces trois composés rendant compte du niveau d'intensité des effets de la pollution sur la peau.

_2.3 Expression des résultats_ :

La quantification de chacun des 3 composés initialement issus de l'ozonolyse du squalène, à savoir le C27-pentaènal, le C22-tetraènal et le C17-triènal, est réalisée pour chaque échantillon à J0 et à J56, et la somme de ces 3 teneurs est nommée « squalène ozonolysé » (S.O.). On évalue ainsi la variation moyenne observée ($\Delta\%$) après deux mois d'utilisation du produit, et exprimée en pourcentage selon la formule :

$$\Delta\% = 100 \text{ x } (S.O. \text{ } J_{56} - S.O. \text{ } J_0) \text{ } / \text{ } S.O. \text{ } J_0,$$

avec S.O. : teneur en squalène ozonolysé ; $J_0$: 1er jour de l'étude ; J56 : cinquante-sixième jour de l'étude.

_2.4 Résultats_ : la variation du squalène ozonolysé ($\Delta\%$) mesurée pour les volontaires ayant appliqué la composition détaillée dans le Tableau 4, s'établie à - 61% et est statistiquement significative selon le test de Student (p< 0,001, t-test).

Ce résultat expérimental montre donc une action protectrice significative inhérente à l'application de la composition comprenant l'extrait A) pour une peau soumise à un agent polluant, en l'occurrence l'ozone.

**3- Formules d'illustration**

**3.1. Spray solaire organo-minéral et anti-pollution**

FORMULE

| | | |
|---|---|---|
| A | Néopentanoate d'isodécyle | 20% |
| | Cyclodiméthicone | 5% |
| | Ethylhexylmethoxicinnamate | 6% |
| | Butyl Methoxydibenzoylmethane | 3% |
| | DL alpha Tocophérol | 0.05% |
| B | Eau | qsp 100% |

(suite)

|   |   |   |
|---|---|---|
| | EDTA tétrasodique | 0,2% |
| | Glycérine | 7% |
| | Phenyl Benzyimidazole Sulfonic Acid (salifié avec quantité molaire nécessaire de soude) | 3% |
| C | SEPIMAX™Zen | 1,3% |
| | Keltrol™CG-T | 0,315% |
| | Efficacia™M | 0,385% |
| D | Extrait A | 1% |
| | SEPICIDE™ HB | 1% |
| | Fragrance | 0,1% |

### 3.2 : Gel-crème Masque visage anti-pollution

<u>FORMULE</u>

|   |   |   |
|---|---|---|
| A | Simmondsia Chinensis seed oil | 14,1% |
| | C12-C15 alkyl Benzoate | 6,7% |
| | Cyclopentasiloxane | 4,2% |
| | DL alpha Tocopherol | 0,10% |
| B | Maris Aqua | qs 100% |
| C | SEPIMAX™Zen | 2% |
| | Keltrol™CG-T | 0,45% |
| | Efficacia™M | 0,55% |
| D | Extrait A | 1,2% |
| | Euxyl PE9010 | 1% |
| | Fragrance | 0,1% |

### 3.3 : Emulsion eau-dans-huile solaire

|   |   |
|---|---|
| Adipate de di-isopropyle | 12% |
| Salicylate d'(éthyl hexyle) | 5% |
| Méthoxycinnamate d'(éthyl hexyle) | 5% |
| 4-(N,N-diméthyl amino) benzoate d'(éthyl hexyle) | 8% |
| Butylméthoxydibenzoylméthane | 2% |
| PEG30 dipolyhydroxystéarate | 0,4% |
| Fluidanov™ 20X | 1,2% |
| Sepimax Zen | 0,7% |
| Glycérine : | 1,5% |
| Euxyl™ PE9010 : | 1% |
| Extrait A) | 1% |
| Eau : | qsp 100% |

### 3-4 : Emulsion eau-dans-huile éclaircissante anti-pollution

|   |   |
|---|---|
| Isononanoate d'isononyle : | 6% |
| Parfum : | 0,1% |

(suite)

| | |
|---|---|
| Phénoxyéthanol & (Ethylhexyl) glycérine : | 1% |
| Easynov™ : | 1,5% |
| SEPIMAX™Zen | 0,5% |
| Sepiwhite™MSH : | 2% |
| Extrait A) | 0,9% |
| Eau : | qsp 100% |

**3-5 : Shampoing colorant anti-pollution**

Formule

| | |
|---|---|
| Phase A | |
| MONTALINE™C40 | 15% |
| Cocoamphoacétate disodé | 5% |
| Cetrimonium chloride | 1% |
| MONTANOV™S | 3% |
| SEPIMAX™ Zen | 1,3 % |
| Phase B | |
| Extrait A) | 1% |
| Couleur | qsp |
| Eau | qsp 100% |

**3-6 : lotion capillaire anti-pollution**

| | |
|---|---|
| Butylène glycol | 3,0% |
| Sepimax™ Zen | 3,0% |
| SIMULSOL™1293 | 3,0% |
| Acide lactique | qs. pH = 6 |
| SEPICIDE™ HB | 0,2% |
| Extrait A) | 1,3% |
| Parfum | 0,3% |
| Eau | qs. 100% |

SEPIMAX™Zen (nom INCI : Polyacrylate Crosspolymer-6) est un agent épaississant, émulsionnant et stabilisant ;
Keltrol™CG-T : Gomme xanthane ;
Efficacia™M est la gomme d'acacia commercialisé par la société CNI
SEPICIDE™ HB (nom INCI : Phenoxyethanol/Methylparaben/Ethylparaben/Propylparaben/ Butylparaben) est un agent conservateur contenant du phénoxyéthanol ;
Euxyl PE9010 (Nom INCI : Phenoxyethanol & Ethylhexyl Glycerin) : composition utilisée comme agent conservateur.
Maris Aqua : eau de mer à 8% de chlorure de sodium.
Fluidanov™ 20X (nom INCI : octydodecanol and octyldodecyl xyloside) est une composition émulsionnante de type eau-dans-huile ;
Sepiwhite™MSH (INCI name : ω-undecylenoyl phenylalanine) est un agent éclaircissant de la peau ;
MONTALINE™C40: (cocamoniumcarbamoyl chloride) ;
MONTANOV™S : (cocoyl glucoside & cocoyl alcohol) ;
SIMULSOL™1293 est de l'huile de castor hydrogénée et éthoxylée, avec un indice d'éthoxylation égal à 40 ;

**EP 3 519 056 B1**

**Revendications**

1. Procédé non-thérapeutique pour empêcher ou ralentir l'apparition sur la peau, le cuir chevelu, les cheveux ou les muqueuses, des signes inesthétiques générés par l'ozone ou bien pour éliminer lesdits signes, ledit procédé comprenant au moins une étape d'application sur la peau humaine, sur les muqueuses, sur le cuir chevelu ou sur les cheveux, d'une formulation cosmétique à usage topique comprenant au moins un excipient cosmétiquement acceptable et entre 0,1% et 5% massique d'un extrait glycolique (EG) d'une biomasse unialgale de cellules de macroalgues pluricellulaires de petite taille issues de l'espèce *Acrochaetium moniliforme,* ledit extrait étant obtenu par le procédé comprenant les étapes successives suivantes :

   - Une étape A) de préparation d'un échantillon unialgal de cellules de macroalgues pluricellulaires, à partir d'un échantillon de macroalgues issues de l'espèce *Acrochaetium moniliforme* et prélevé dans le milieu ;
   - Une étape B) de mise en culture dudit échantillon unialgal de cellules de macroalgues pluricellulaires obtenu à l'étape A) dans un photobioréacteur contenant de l'eau de mer additionnée de nitrate de sodium (NaNO$_3$) à une concentration comprise entre 50 mg/dm$^3$ et 250 mg/dm$^3$ et de dihydrogénophosphate de sodium (NaH$_2$PO$_4$) à une concentration comprise entre 5 mg/dm$^3$ et 75 mg/dm$^3$ et à une température comprise entre 10°C et 25°C, pour obtenir une suspension aqueuse de ladite biomasse unialgale de cellules de macroalgues pluricellulaires de petite taille ;
   - Une étape C) de récolte de ladite biomasse unialgale de cellules de macroalgues pluricellulaires de petite taille à partir de ladite suspension aqueuse obtenue à l'issue de l'étape B) au moyen d'une toile filante au seuil de coupure compris entre 25µm et 100µm puis pressage de la biomasse obtenue pour éliminer l'eau résiduelle ;
   - une étape D) de préparation d'une poudre de ladite biomasse unialgale de cellules de macro-algues pluricellulaires de petite taille obtenue à l'étape C), par congélation de ladite biomasse puis sa lyophilisation son broyage ;
   - Une étape E) au cours de laquelle la poudre de ladite biomasse à l'étape D), est dispersée sous agitation dans un mélange eau-1,3-butanediol dans lequel le ratio volumique eau sur 1,3-butanediol est inférieur ou égal à 1/1 et supérieur ou égal à 1/9, pour former une dispersion de ladite biomasse dans ledit mélange eau-1,3-butanediol comprenant pour 100% de sa masse, de 1% massique à 20% massique deladite biomasse; et
   - Une étape F) au cours de laquelle la dispersion obtenue à l'étape E) précédente, est séparée en ses phases non miscibles, pour recueillir ledit extrait glycolique (EG).

2. Procédé non-thérapeutique tel que défini dans la revendication 1, pour lequel lesdits signes inesthétiques générés par l'ozone, sont les rougeurs, les rides, les ridules ou l'altération du microrelief de la peau, du cuir chevelu ou des muqueuses, le ternissement du teint de la peau ou encore le ternissement des cheveux et/ou de leur fragilisation vis-à-vis des contraintes mécaniques.

**Patentansprüche**

1. Ein nicht-therapeutisches Verfahren zum Verhindern oder Verlangsamen des Auftretens von durch Ozon erzeugten unästhetischen Anzeichen auf der Haut, der Kopfhaut, den Haaren oder den Schleimhäuten, oder auch zum Beseitigen der genannten Anzeichen, wobei das genannte Verfahren mindestens einen Schritt des Auftragens einer kosmetischen Formulierung zur topischen Anwendung auf die menschliche Haut, die Schleimhäute, die Kopfhaut oder die Haare umfasst, die mindestens einen kosmetisch verträglichen Hilfsstoff und zwischen 0,1 % und 5 % Massenanteil eines glykolischen Extrakts (EG) einer einalgigen Biomasse von Zellen von mehrzelligen Makroalgen kleiner Größe aus der Spezies *Acrochaetium moniliforme* umfasst, wobei der genannte Extrakt durch das Verfahren erhalten wird, das die folgenden aufeinanderfolgenden Schritte umfasst:

   - Ein Schritt A) der Herstellung einer einalgigen Probe von Zellen von mehrzelligen Makroalgen, ausgehend von einer Probe von Makroalgen aus der Spezies Acrochaetium moniliforme, die im Medium entnommen wurde;
   - Ein Schritt B) der Kultivierung der genannten einalgigen Probe von Zellen von mehrzelligen Makroalgen, die in Schritt A) erhalten wurde, in einem Photobioreaktor, der mit Natriumnitrat (NaNO$_3$) in einer Konzentration zwischen 50 mg/dm$^3$ und 250 mg/dm$^3$ und Natriumdihydrogenphosphat (NaH$_2$PO$_4$) in einer Konzentration zwischen 5 mg/dm$^3$ und 75 mg/dm$^3$ und bei einer Temperatur zwischen 10°C und 25°C angereichertes Meerwasser enthält, um eine wässrige Suspension der genannten einalgigen Biomasse von Zellen von mehrzelligen Makroalgen kleiner Größe zu erhalten;
   - Ein Schritt C) der Ernte der genannten einalgigen Biomasse von Zellen von mehrzelligen Makroalgen kleiner Größe aus der genannten wässrigen Suspension, die am Ende von Schritt B) erhalten wurde, mittels eines Filtertuchs mit einer Trennschwelle zwischen 25 μm und 100 μm, gefolgt vom Pressen der erhaltenen Biomasse, um das Restwasser zu entfernen;
- ein Schritt D) der Herstellung eines Pulvers der genannten einalgigen Biomasse von Zellen von mehrzelligen Makroalgen kleiner Größe, die in Schritt C) erhalten wurde, durch Einfrieren der genannten Biomasse, dann deren Lyophilisierung, deren Mahlen;
- Ein Schritt E), in dessen Verlauf das Pulver der genannten Biomasse aus Schritt D) unter Rühren in einem Wasser-1,3-Butandiol-Gemisch dispergiert wird, in dem das Volumenverhältnis von Wasser zu 1,3-Butandiol kleiner oder gleich 1/1 und größer oder gleich 1/9 ist, um eine Dispersion der genannten Biomasse in dem genannten Wasser-1,3-Butandiol-Gemisch zu bilden, das für 100 % seiner Masse von 1 % Massenanteil bis 20 % Massenanteil der genannten Biomasse umfasst; und
- Ein Schritt F), in dessen Verlauf die im vorhergehenden Schritt E) erhaltene Dispersion in ihre nicht mischbaren Phasen getrennt wird, um den genannten glykolischen Extrakt (EG) zu gewinnen.

**2.** Das nicht-therapeutische Verfahren nach Anspruch 1, wobei die genannten durch Ozon erzeugten unästhetischen Anzeichen Rötungen, Falten, Fältchen oder die Veränderung des Mikroreliefs der Haut, der Kopfhaut oder der Schleimhäute, die Mattigkeit des Teints der Haut oder auch die Mattigkeit der Haare und/oder deren Brüchigwerden gegenüber mechanischen Belastungen sind.


**Claims**

**1.** A non-therapeutic method for preventing or slowing down the appearance on the skin, scalp, hair or mucous membranes, of unaesthetic signs generated by ozone or else for eliminating said signs, said method comprising at least one step of application to human skin, mucous membranes, scalp or hair, of a cosmetic formulation for topical use comprising at least one cosmetically acceptable excipient and between 0.1% and 5% by mass of a glycolic extract (EG) of a unialgal biomass of small-sized multicellular macroalgae cells from the species Acrochaetium moniliforme, said extract being obtained by the method comprising the following successive steps:

- A step A) of preparing a unialgal sample of multicellular macroalgae cells, from a sample of macroalgae from the species *Acrochaetium moniliforme* and collected in the medium;
- A step B) of culturing said unialgal sample of multicellular macroalgae cells obtained in step A) in a photobioreactor containing seawater supplemented with sodium nitrate ($NaNO_3$) at a concentration comprised between 50 mg/dm$^3$ and 250 mg/dm$^3$ and sodium dihydrogen phosphate ($NaH_2PO_4$) at a concentration comprised between 5 mg/dm$^3$ and 75 mg/dm$^3$ and at a temperature comprised between 10°C and 25°C, to obtain an aqueous suspension of said unialgal biomass of small-sized multicellular macroalgae cells;
- A step C) of harvesting said unialgal biomass of small-sized multicellular macroalgae cells from said aqueous suspension obtained at the end of step B) by means of a filter cloth with a cut-off threshold comprised between 25μm and 100μm then pressing the biomass obtained to eliminate the residual water;
- a step D) of preparing a powder of said unialgal biomass of small-sized multicellular macroalgae cells obtained in step C), by freezing said biomass then its lyophilization its grinding;
- A step E) during which the powder of said biomass from step D) is dispersed under agitation in a water-1,3-butanediol mixture in which the volume ratio of water to 1,3-butanediol is less than or equal to 1/1 and greater than or equal to 1/9, to form a dispersion of said biomass in said water-1,3-butanediol mixture comprising for 100% of its mass, from 1% by mass to 20% by mass of said biomass; and
- A step F) during which the dispersion obtained in the preceding step E) is separated into its non-miscible phases, to collect said glycolic extract (EG).

**2.** The non-therapeutic method according to claim 1, wherein said unaesthetic signs generated by ozone are redness, wrinkles, fine lines or the alteration of the microrelief of the skin, scalp or mucous membranes, the dullness of the skin's complexion or else the dullness of the hair and/or its embrittlement with respect to mechanical stresses.

**EP 3 519 056 B1**

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 557042 A1
- EP 1230914 A1
- KR 101409764
- WO 2013178965 A2
- FR 2911278 A1
- KR 101128591 B1
- US 2006198800 A1
- CN 103858745 A
- CN 103931482 A
- EP 0971683 A
- EP 1515688 A2
- WO 9600719 A

**Littérature non-brevet citée dans la description**

- **E. DRAKAKI** ; **C. DESSINIOTI** ; **C. V. ANTONIOU**. *Frontiers in Environmental Science*, 2014, vol. 2, 1-6
- **MA LEFEBVRE** ; **DM PHAM** ; **B BOUSSOUIRA** ; **D BERNARD** ; **C CAMUS** ; **QL NGUYEN**. *International Journal of Cosmetic Science*, 2015, vol. 37, 329-38
- **MA LEFEBVRE** ; **DM PHAM** ; **B BOUSSOUIRA** ; **H QIU** ; **C YE** ; **X LONG** ; **R CHEN** ; **W GU** ; **A LAURENT** ; **QL NGUYEN**. *International Journal of Cosmetic Science*, 2016, vol. 38, 217-23
- **A. VIERKÖTTER**. *Hautarzt*, 2010, vol. 61, 538-9
- **YS YANG** ; **HK LIM** ; **KK HONG** ; **MK SHIN** ; **JW LEE** ; **SW LEE** ; **NI KIM**. *Annals of Dermatology*, 2014, vol. 26, 11-16
- **S. E. MANCEBO** ; **S. Q. WANG**. *Journal of European Academy of Dermatology and Venerology*, 2015, vol. 29, 2326-2332
- **ND MAGNANI** ; **XM MURESAN** ; **G BELMONTE** ; **F CERVELLATI** ; **C STICOZZI** ; **A PECORELLI** ; **C MIRACCO** ; **T MARCHINI** ; **P EVELSON** ; **G VALACCHI**. *Toxicological Sciences*, 2016, vol. 149, 227-36
- **RONELIE C et al.** Non-enzymatic isolation of somatic cells from Kappaphycus spp. and Eucheuma denticulatum (Solieriaceae, Rhodophyta). *Eur. J. Phycol*, 2014, vol. 49 (4), 486-492
- **CLINTON J. DAWES et al.** Branch, micropropagule and tissue culture of the red algae Eucheuma denticulatum and Kappaphycus alvarezii farmed in the Philippines. *Jouanl of applied Phycology*, 1991, vol. 3, 247-257
- **MYOUNGHOON et al.** Isolation and charactrization of thermostable phycocyanin from Galdieria sulphuraria. *Korean J. Chem. Eng*, vol. 31 (3), 490-495
- Production of bioactive metabolites by cell and tissue cultures of marine macroalgae in bioreactor systems. **GREGORY L. RORRER et al.** Plant Cell and Tissue Culture for the Production of Food Ingredients. Kluwer Academic / Plenum Publishers, 1999
- **C. ZHI** ; **G. L. RORRER**. Photolithotrophic cultivation of Laminaria saccharina gametophyte cells in a bubble-column bioreactor. *Enzyme and Microbial Technology*, March 1996, vol. 18 (4), 291-299
- **H. STEGENGA et al.** Remarks on the Audouinella microscopica (NÄG.) Woekerling complex, with a brief survey of the genus chromastrum papenfuss (Rhodophyta, Nemaliales). *Acta Bot. Neerl*, August 1979, vol. 28 (4/5), 289-311
- **RONELIE C et al.** Nonenzymatic isolation of somatic cells from Kappaphycus spp. and Eucheuma denticulatum (Solieriaceae, Rhodophyta). *Eur. J. Phycol*, 2014, vol. 49 (4), 486-492
- **J. MUNOZ**. Use of plant growth regulators in micropropagation of Kappaphycus alvarezii (Doty) in airlift bioreactors. *J Appl Phycol*, 2006, vol. 18, 209-218
- **S. MALIAKAL** ; **D. CHENEY**. Halogenated monoterpenes production in regenerated plantlet cultures of Ochtodes secundiramea. *J. Phycol*, 2001, vol. 37, 1010-1019
- **A RUSIG** ; **J. COSSON**. Plant regeneration from protoplasts of Enteromorpha intestinalis (Chlorophyta, Ulvophyceae) as seedstock for macroagal culture. *Journal of Applied Phycology*, 2001, vol. 13, 103-108
- **A. WHISTHALER** ; **C.J. WESCHLER**. *PNAS*, 13 April 2010, vol. 107 (15), 6568-6575